(19) Europäisches Patentamt
European Patent Office
Office européen des brevets

(11) Numéro de publication: 0 004 810 B1

# (12) FASCICULE DE BREVET EUROPEEN

(45) Date de publication du fascicule du brevet: **23.04.86**

(51) Int. Cl.⁴: **A 61 K 31/38,** A 61 K 7/48 // C07D333/38

(21) Numéro de dépôt: **79400198.2**

(22) Date de dépôt: **28.03.79**

(54) **Le peroxyde de thénoyle à titre de médicament ainsi que son application à titre de cosmétique, compositions pharmaceutiques et compositions cosmétiques le renfermant.**

(30) Priorité: **31.03.78 FR 7809616**

(43) Date de publication de la demande: **17.10.79 Bulletin 79/21**

(45) Mention de la délivrance du brevet: **23.04.86 Bulletin 86/17**

(84) Etats contractants désignés: **BE CH DE FR GB LU NL SE**

(56) Documents cités:
**BE - A - 687 356**
**FR - A - 2 043 477**
**FR - M - 6 698**

(73) Titulaire: **ROUSSEL-UCLAF, 102, route de Noisy Boîte postale no.9, F-93230 Romainville (FR)**

(72) Inventeur: **Bonne, Claude, 12 bis avenue Aristide Briand, F-94360 Bry-sur-Marne (FR)**
Inventeur: **Marchandeau, Christian, 1, rue de Dovy, Annet-sur-Marne, F-77410 Claye-Souilly (FR)**

(74) Mandataire: **Douetteau, Pierre et al, c/o ROUSSEL-UCLAF 102, route de Noisy Boite postale 9, F-93230 Romainville (FR)**

**EP 0 004 810 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne le peroxide de thénoyle pour son utilisation dans une méthode de traitement thérapeutique du corps humain ou animal, les compositions pharmaceutiques le renfermant ainsi que l'application du produit à usage cosmétique et les compositions cosmétiques le renfermant.

Le peroxyde de thénoyle de formule:

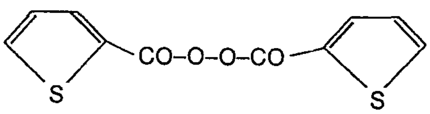

a déjà été décrit en tant que produit chimique; il a notamment été utilisé dans la polymérisation du styrène (MONATS, 1949, 80, 739), mais jusqu'alors aucune propriété pharmacologique ni aucune application thérapeutique n'avaient été signalées pour ce produit.

Or les études menées par la société demanderesse viennent de mettre en évidence les propriétés anti-bactérienne et comédolytique remarquables de ce produit.

L'invention a ainsi pour objet le composé chimique caractérisé en ce qu'il est constitué par le peroxyde de thénoyle pour son utilisation dans une méthode de traitement thérapeutique du corps humain ou animal, notamment pour son utilisation dans une méthode de traitement de l'acné.

Le peroxyde de thénoyle est destiné notamment au traitement de l'acné lorsque les lésions (comédons et pustules) sont installées.

L'invention a également pour objet les compositions pharmaceutiques renfermant le peroxyde de thénoyle à titre de principe actif, ainsi qu'un excipient pharmaceutique inerte.

Parmi les compositions de l'invention, on retient notamment celles qui renferment de 2 à 20% de principe actif.

Les compositions ci-dessus sont plus particulièrement réalisées de manière à pouvoir être administrées par voie locale et pour cela peuvent se présenter sous forme de solutions, d'émulsions, de crèmes, de pommades, de poudres, de lotions et de gels.

Les formes pharmaceutiques sont préparées selon les méthodes usuelles. Le principe actif peut y être incorporé à des excipients habituellement employés dans ces compositions, notamment, des polyéthylèneglycols, des cires, des corps gras, des dérivés stéariques, du talc, des alcools, tels que les alcools stéaryliques, cétostéaryliques, des huiles végétales, telles que l'huile d'amande douce, des huiles minérales, des mouillants, des épaississants, des conservateurs, des parfums, des colorants ou d'autres excipients connus. Bien entendu, certains excipients qui pourraient réagir de façon indésirable avec le peroxyde de thénoyle doivent être évités. Il en est ainsi, par exemple des corps gras contenant des insaturations.

La dose usuelle, variable selon le sujet à traiter et l'affection en cause, peut être par exemple de 1 à 4 applications par jour, d'une pommade renfermant de 2 à 20% de principe actif, chez l'homme.

Le peroxyde de thénoyle peut également être utilisé dans les usages cosmétiques et l'invention a ainsi également pour objet l'application du peroxyde de thénoyle à titre de produit cosmétique.

Il est alors plus particulièrement destiné à l'entretien de la peau.

L'invention a également pour objet les compositions cosmétiques renfermant le peroxyde de thénoyle.

Parmis ces compositions, on retient notamment celles qui renferment de 0,2 à 20% de peroxyde de thénoyle.

Les compositions cosmétiques de l'invention sont destinées essentiellement à être appliquées sur la peau et peuvent, par exemple, se présenter sous forme de solution, d'émulsions, de crèmes, de pommades, de poudres, de lotions, de gels, de savons, de laits, de masques, d'aérosols ou d'huile pour le bain.

Ces compositions cosmétiques peuvent être préparées selon les méthodes usuelles. Les constituants autres que le peroxyde de thénoyle sont analogues à ceux entrant dans la formule des compositions pharmaceutiques mentionnées ci-dessus.

Il est à noter qu'un produit proche du peroxyde de thénoyle, le peroxyde de benzoyle, est utilisé chez l'homme pour le traitement de l'acné, cependant, le peroxyde de benzoyle présente des difficultés d'utilisation dues à son instabilité. En effet, ce produit explose facilement à l'état sec à une température de 70–80 °C et les préparations pharmaceutiques le contenant se dégradent souvent au stockage (J. Pharm. Sci. 1977, 66, 718).

Le peroxyde de thénoyle présente, quant à lui, une vitesse de décomposition spontanée mesurée à 75 °C deux fois inférieure à celle du peroxyde de benzoyle (J. Org. Chem. 1962, 27, 410) et il ne se décompose de façon explosive qu'au dessus de 130 °C.

Il avait été démontré (Canad. Med. Ass. J. 1965, 93, 252) que les propriétés du peroxyde de benzoyle étaient liées au pouvoir peroxydant de ce produit, par libération lente d'oxygène après application sur la peau.

On aurait donc pu s'attendre à ce que le peroxyde de thénoyle ait une activité pharmacologique bien moindre que celle du peroxyde de benzoyle puisqu'il est plus stable que ce dernier.

Or, la société demanderesse a constaté que, de façon inattendue, le peroxyde de thénoyle est au moins aussi actif que le peroxyde de benzoyle.

Le peroxyde de thénoyle constitue donc, grâce à sa grande stabilité, un produit présentant des avantages très importants sur le peroxyde de benzoyle utilisé jusqu'alors.

Ces avantages sont montrés par les tests rapportés ci-après qui illustrent les propriétés du produit de l'invention.

ETUDE PHARMACOLOGIQUE DU PEROXYDE DE THENOYLE:

1) Activité antibactérienne et antifongique

Les concentrations minimales inhibitrices (C.M.I.) ont été obtenues par la méthode des dilutions sériées en milieu liquide pour Propionibacterium Acnes et en milieu gélosé pour Pityrosporum ovale.

| PRODUITS | C.M.I. (µg/ml) PROPIONIBACTERIUM ACNES | PITYROSPORUM OVALE |
|---|---|---|
| Peroxyde de thénoyle | 12,5 à 37,5 | 250 |
| Peroxyde de benzoyle | 25 à 50 | – |

2) Activité comédolytique

Des groupes de 3 souris femelles atrichis «rhinocéros» présentant spontanément des comédons reçoivent quotidiennement une application cutanée de 0,1 $cm^3$ d'acétone contenant du peroxyde de thénoyle en solution à 10%. Cette application est répartie sur 1 $cm^2$ environ. Le groupe témoin reçoit le solvant dans les mêmes conditions. Le traitement comporte 15 applications. 24 heures après la dernière, une large biopsie est faite dans la zone traitée et fixée dans une solution de BOUIN.

Les biopsies sont ensuites coupées à la paraffine à 5 µ perpendiculairement à la surface cutanée et colorées à l'hémalunéosine.

Les mesures histologiques sont alors effectuées pour 30 comédons pris au hasard dans chaque groupe. Le diamètre de l'orifice (d) et le grand diamètre (D) sont déterminés comme indiqué sur le schéma ci-dessous:

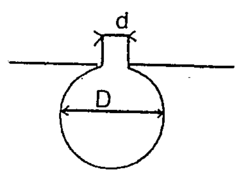


lorsque la coupe ne passe pas par l'orifice du comédon, (d) est défini comme égal à zéro.

Le nombre de comédons mesurés est suffisamment important pour annuler l'influence de l'incidence de coupe dans la comparaison des rapports d/D pour les différents groupes.

RESULTATS: Mesure de l'orifice et du grand diamètre des comédons:

| TRAITEMENT | d* | D* | Rapport d/D |
|---|---|---|---|
| Témoins | 8,7±1,5 | 25 ±2,2 | 0,35 |
| Peroxyde de thénoyle | 21,1±1,7 | 23,5±1,6 | 0,90 |
| Peroxyde de benzoyle | 14,7±2,1 | 27,0±1,8 | 0,54 |

* exprimés en unités arbitraires tenant compte des grossissements de l'objectif et du système écran sur lequel est faite la mesure.

Ces résultats montrent que, chez les témoins, le comédon moyen présente un orifice plus petit que celui des animaux traités par le peroxyde de tnénoyle alors que le grand diamètre est inchangé. Cette évolution du rapport d/D vers la valeur 1, c'est-à-dire la transformation des comédons clos en comédons ouverts, permet l'extrusion du matériel corné comédonien, phénomène fréquemment observé à l'examen microscopique.

EXEMPLE DE PRÉPARATION GALENIQUE:

On a préparé une pommade pour application topique, contenant:

| | |
|---|---|
| Peroxyde de thénoyle | 10 g |
| Alcool éthylique | 40 g |
| Ether polyglycolique d'alcools gras saturés | 10 g |
| (Carbopol 940 (polymère carboxyvinylique) | |
| (diisopropanolamine | |
| sel disodique de l'EDTA | |
| eau | q.s.p 100 g). |

Exemples de compositions cosmétiques

1) On a préparé un gel pour application topique, contenant:

| | |
|---|---|
| Peroxyde de thénoyle | 10 g |
| Excipient | q.s.p. 100 g. |

(Détail de l'excipient: carboxypolyméthylène, triéthanolamine, alcool éthylique, polyoxyéthylène 2,3-lauryléther, propylène glycol, eau).

2) On a préparé un gel pour application topique, contenant:

| | | |
|---|---|---|
| Peroxyde de thénoyle | | 5 g |
| Excipient | q.s.p. | 100 g. |

(Détail de l'excipient: hydroxypropylméthylcellulose et silicate, hydroxyde de sodium, polyoxyéthylène 4-lauryléther, propylène glycol, eau).

**Revendications**

1. Composé chimique constitué par le peroxyde de thénoyle de formule

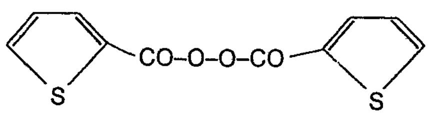

pour son utilisation dans une méthode de traitement thérapeutique du corps humain ou animal.

2. Composé chimique selon la revendication 1 pour son utilisation comme antibactérien.

3. Composé chimique selon la revendication 1 pour son utilisation comme antifongique.

4. Composé chimique selon la revendication 1 pour son utilisation comme comédolytique.

5. Composé chimique selon la revendication 1 pour son utilisation dans le traitement de l'acné.

6. Compositions pharmaceutiques caractérisées en ce qu'elles renferment, à titre de principe actif, le peroxyde de thénoyle, ainsi qu'un excipient pharmaceutique inerte.

7. Compositions selon la revendication 6, caractérisées en ce qu'elles renferment de 2 à 20% de principe actif.

8. Application à titre de produit cosmétique du peroxyde de thénoyle.

9. Compositions cosmétiques, caractérisées en ce qu'elles renferment le peroxyde de thénoyle.

10. Compositions selon la revendication 9, caractérisées en ce qu'elles renferment de 0,2 à 20% de peroxyde de thénoyle.

**Claims**

1. Chemical compound composed of thenoyl peroxide with the formula

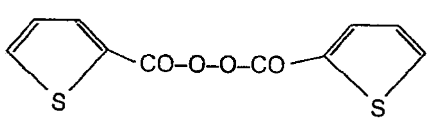

for its use in a method of therapeutic treatment for the human or animal body.

2. Chemical compound according to claim 1 for its antibacterial use.

3. Chemical compound according to claim 1 for its antifungal use.

4. Chemical compound according to claim 1 for its comedolytic use.

5. Chemical compound according to claim 1 for its use in the treatment of acne.

6. Pharmaceutical compositions characterized in that they contain, as active principle, thenoyl peroxide, as well as an inert pharmaceutical excipient.

7. Compositions according to claim 6, characterized in that they contain from 2 to 20% of active principle.

8. Use as a cosmetic product of thenoyl peroxide.

9. Cosmetic compositions, characterized in that they contain thenoyl peroxide.

10. Compositions according to claim 9, characterized in that they contain from 0.2 to 20% of thenoyl peroxide.

**Patentansprüche**

1. Chemische Verbindung bestehend aus Thenoylperoxid der Formel

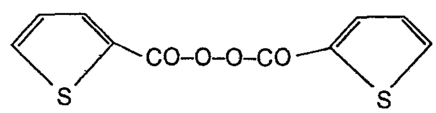

zur Anwendung in einem Verfahren zur therapeutischen Behandlung des menschlichen oder tierischen Körpers.

2. Chemische Verbindung gemäss Anspruch 1 zur Anwendung als antibakterielle Verbindung.

3. Chemische Verbindung gemäss Anspruch 1 zur Anwendung als gegenüber Fungi wirkende Verbindung.

4. Chemische Verbindung gemäss Anspruch 1 zur Anwendung als comedolytische Verbindung.

5. Chemische Verbindung gemäss Anspruch 1 zur Anwendung bei der Behandlung von Akne.

6. Pharmazeutische Zusammensetzungen, dadurch gekennzeichnet, dass sie als Wirkstoff Thenoylperoxid sowie einen inerten pharmazeutischen Exzipienten enthalten.

7. Zusammensetzungen gemäss Anspruch 6, dadurch gekennzeichnet, dass sie 2 bis 20% Wirkstoff enthalten.

8. Verwendung von Thenoylperoxid als kosmetisches Produkt.

9. Kosmetische Zusammensetzungen, dadurch gekennzeichnet, dass sie Thenoylperoxid enthalten.

10. Zusammensetzungen gemäss Anspruch 9, dadurch gekennzeichnet, dass sie 0,2 bis 20% Thenoylperoxid enthalten.